# EUROPEAN PATENT APPLICATION

(11) **EP 3 744 269 A1**
(43) Date of publication of application: **02.12.2020**
(21) Application number: 18902052.2
(22) Date of filing: 13.09.2018
(51) Int. Cl.: A61B 17/34, A61M 5/34, A61B 17/00

(54) **CANNULA HAVING EXCELLENT NEEDLE COUPLING FORCE**

(30) Priority: 23.01.2018 KR 20180000337 U
(71) Applicant: Kovec-Med Co., Ltd., Gyeonggi-do 15608 (KR)
(72) Inventor: IN, Han-jin, Seoul 06294 (KR)
(74) Representative: Spengler, Robert
(86) International application number: PCT/KR2018/010795
(87) International publication number: WO 2019/146867

(57) **Abstract**

The present invention relates to a cannula having an excellent coupling force and fixing force between an expansion part and a needle and, more specifically, the needle is formed in a cylinder shape having a predetermined length, and includes a coupling means, which is formed at the outer surface thereof, and is inserted into the expansion part such that the coupling force can increase by the friction with the expansion part during adhesion, wherein the coupling means includes: a groove part formed, in an insertion region of the needle inserted into the expansion part, by the melting of the outer surface of the needle through laser machining so as to have a predetermined depth; and a protrusion part protruding in the outward direction of the needle so as to be connected to the groove part.

## Description

### [Technical Field]

The present invention relates to a cannula having excellent coupling force and fixing force between an expansion portion and a needle.

### [Background Art]

In general, a cannula is widely used in the form of a needle necessary for collecting blood of a patient as well as for liposuction or injection of a filler, and a needle having a smooth surface is inserted into a hollow portion formed in an expansion portion in an interference fitting fashion, wherein an adhesive is applied to the surface of the needle in order to increase coupling force or fixing force with the expansion portion.

For example, in the case of a conventional liposuction cannula disclosed in Korean Registered Patent No. 10-1472943 shown in FIG. 1, in order to insert a laser treatment line for liposuction or to insert an aspirator for liposuction, the laser treatment line or the aspirator is inserted in the state in which the liposuction cannula is inserted into a medical treatment region, such as an abdominal cavity.

Next, in the case of a filler injection cannula used for injecting a filler, the filler injection cannula is inserted into a skin layer to inject a filler into a region such as facial wrinkles, and the filler injection cannula is removed in the state in which only the filler is left such that the skin layer is filled with the filler. Medical treatment is generally performed using the same cannula although the use thereof is different.

Meanwhile, unlike the liposuction cannula and filler injection cannula described above, a skin wrinkle treatment cannula is used in the case in which medical yarn is buried in a skin layer in order to remove the skin wrinkles or to accelerate the supply of nutrients to the skin, thereby increasing elasticity of the skin.

Such a cannula for medical use is generally configured such that an expansion portion 3 and a needle 10 are adhered to each other via an adhesive (not shown). An injector or an aspirator formed in the shape of a syringe to the expansion portion, and liposuction or filler injection is performed through a tubular needle. In recent years, with growth of medical equipment, the injector or the aspirator has been realized in the form of a device, whereby rapid medical treatment is possible. At the time of liposuction or filler injection, predetermined discharge/suction pressure is present in the medical equipment.

In the case in which a needle having a smooth surface and an expansion portion having a smooth inner wall of a hollow portion are adhered to each other using an adhesive, however, friction force therebetween is low, whereby a safety-related accident in which the needle is separated from the expansion portion may occur.

### [Disclosure]

### [Technical Problem]

The present invention has been made in view of the above problems, and it is an object of the present invention to provide technology capable of increasing frictional force between an expansion portion and a needle inserted into the expansion portion, thereby increasing coupling force and fixing force.

### [Technical Solution]

In accordance with an aspect of the present invention, the above and other objects can be accomplished by the provision of a cannula having excellent coupling force of a needle, wherein the needle is formed so as to have a cylindrical tubular shape or a cylindrical shape having a predetermined length, and includes a coupling means formed at the outer surface thereof to increase coupling force due to frictional force with the expansion portion when the needle is inserted into and adhered to the expansion portion, and wherein the coupling means includes a groove portion formed by melting the outer surface of the needle through laser machining so as to have a predetermined depth in an insertion region of the needle, which is inserted into the expansion portion, and a protruding portion protruding in the outward direction of the needle so as to be connected to the groove portion.

In addition, the coupling means may further include an auxiliary fixture formed on the outer surface of the needle so as to be adjacent to the groove portion while having a nonspecific pattern, the auxiliary fixture being formed as the result of melting generated at the time of laser machining of the groove portion being fixed to the outer surface of the needle.

In addition, the groove portion may have a depth of 1 to 60 µm and a width of 20 to 100 µm.

In addition, the protruding portion has a height of 1 to 30 µm.

In accordance with another aspect of the present invention, there is provided a cannula for medical treatment configured such that an expansion portion and a needle are adhered to each other via an adhesive, the cannula having excellent coupling force of the needle, wherein the needle is formed so as to have a cylindrical tubular shape or a cylindrical shape having a predetermined length, and includes a coupling means formed at the outer surface thereof to increase coupling force due to frictional force with the expansion portion when the needle is inserted into and adhered to the expansion portion, and wherein the coupling means is configured such that a laser is applied to the outer circumferential face of the entirety or a portion of the needle inserted into the expansion portion in a point shot fashion, whereby the surface of the needle to which the laser is applied has a rough texture.

### [Advantageous effects]

According to the present invention, the needle, configured such that the adhesive is applied over the insertion region in which the coupling means including the groove portion and the protruding portion and further including the auxiliary fixture is formed, is inserted into the expansion portion, whereby the present invention has the effect of increasing coupling force and fixing force between the expansion portion and the needle due to frictional force of the coupling means, unlike the conventional art.

### [Description of Drawings]

FIG. 1 is a view showing a conventional cannula.
FIG. 2 is a view of a cannula having excellent coupling force of a needle according to the present invention.
FIG. 3 is an exploded cross-sectional view of FIG. 2.
FIG. 4 is an enlarged cross-sectional view of FIG. 3.
FIG. 5 is an enlarged photograph showing the cannula having excellent coupling force of the needle according to the present invention.
FIG. 6 is an actual photograph showing the state in which the cannula having excellent coupling force of the needle according to the present invention is aligned on a laser processing apparatus.
FIG. 7 is a view showing another embodiment of a coupling means of the cannula having the excellent coupling force of the needle according to the present invention.

### [Best Mode]

Hereinafter, a cannula having excellent coupling force of a needle (hereinafter simply referred to as a "cannula") according to a preferred embodiment of the present invention will be described in detail with reference to the accompanying drawings. Prior to description, in the present invention, an expansion portion 3 means a general expansion portion 3 which is made of a synthetic resin material, such as transparent or opaque silicone, into one side of which a needle 10, a description of which will follow, is inserted, and to the other side of which a syringe needle, such as an aspirator or an injector, and an acupuncture-needle-shaped patient blood collection needle having a sharp end is connected, and a detailed description thereof will be omitted in order to make clear the gist of the present invention.

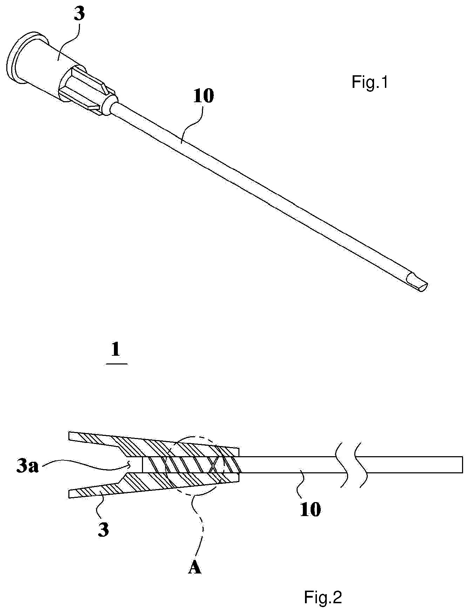

First, as shown in FIG. 2, a cannula 1 according to the present invention mainly includes an expansion portion 3 and a needle 10.

More specifically, the needle 10 may be formed so as to have a cylindrical tubular shape (a needle-like shape) that is open at opposite cylindrical sides thereof or to have a cylindrical shape (an acupuncture-needle-like shape), and may include a coupling means 100 provided at the outer surface thereof to increase coupling force and fixing force (necessary to prevent unnecessary rotation of the needle) when the needle is inserted into the expansion portion 3 and is fixed by adhesion using an adhesive G.

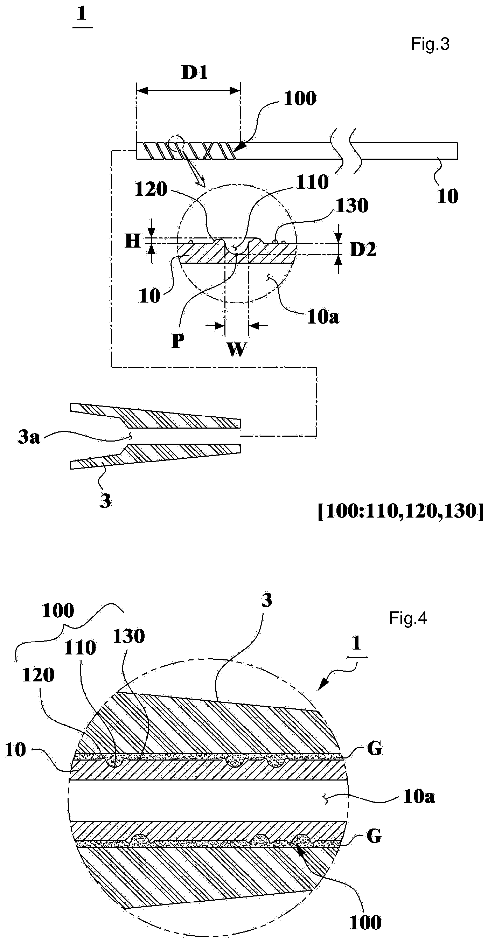

For example, as shown in FIG. 3, the coupling means 100 may include a groove portion 110 and a protruding portion 120, and a laser is applied to the outer surface of the needle 10 in order to melt the surface of the needle 10 and thus to form the coupling means 100.

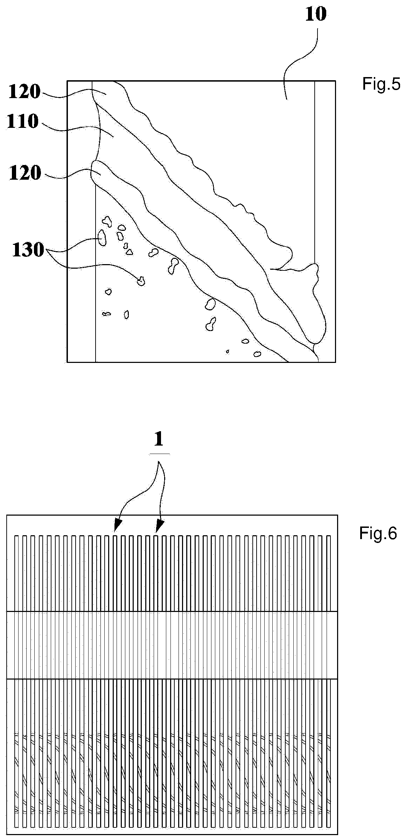

At this time, preferably, a plurality of needles 10 is arranged in a line on a laser processing apparatus (not shown), and a laser is applied perpendicularly to the surface of each of the needle in order to form the coupling means 100 at the surface of each of the needles, or each of the needles 10 that are arranged is rotated such that the coupling means 100 is formed along the outer circumferential surface of each of the needles as needed, whereby it is possible to minimize processing time (see FIG. 6).

Here, the groove portion 110 may be formed in an insertion region D1 of the needle 10 while being formed in one surface of the needle 10, or may be formed along the outer circumferential surface of the needle 10 so as to have a predetermined angle without a specific pattern in order to further increase the coupling force of the needle.

At this time, preferably, the insertion region D1 of the needle 10 has a length within 1 to 200 mm depending on the length from the front end to the rear end of the needle 10.

In addition, a plurality of groove portions 110 is formed within the insertion region D1, and in the present invention, the insertion region D1 means the insertion length of the needle 10 that is inserted into the expansion portion 3.

At this time, preferably, the depth D2 of the groove portion 110 formed as the result of being melted by the applied laser is limited to 1 to 60 µm or less, and the width W of the groove portion is limited to 20 to 100 µm or less. The reason for this is that, in the case in which the depth D2 of the groove portion 110 is less than the limited depth, the height of the protruding portion 120, a description of which will follow, is low and thus frictional force with the expansion portion 3 is decreased, whereby coupling force is reduced, or the amount of the adhesive G that is introduced into the groove portion 110 is small at the time of applying the adhesive G, whereby it is difficult to increase coupling force at the time of coupling with the expansion portion 3, and that the depth of the groove portion is greater than the limited depth, the cross section between the lowest bottom point P of the groove portion 110 and an injection path 10a formed in the needle 10 in the longitudinal direction thereof becomes thin, although the height of the protruding portion 120 is increased, whereby damage to (breakage of) the needle 10 may occur easily.

In addition, referring to FIG. 4, the reason to limit the width of the groove portion 110 is that in the case in which the groove portion has a width less than the limited width, it takes a long time for the adhesive G, which has high viscosity, to be introduced into the groove portion 110, whereby the processing time necessary to apply the adhesive G may be increased, and that, in the case in which the groove portion has a width greater than the limited width, the strength of the needle 10 in the insertion region D1 is reduced when a plurality of groove portions 110 is formed in the insertion region D1, although the processing time necessary to apply the adhesive G is decreased, whereby the needle may be damaged.

In addition, the protruding portion 120 is formed so as to protrude as the result of the needle 10 being melted when a laser is applied to the outer surface of the needle 10 in order to form the groove portion 110, is provided to increase frictional force between the needle and the inner wall of a hollow portion 3a of the expansion portion 3, and is formed in a protrusion fashion so as to have a limited height H within 1 to 30 µm or less.

The reason that the height of the protruding portion 120 is limited is that, in the case in which the protruding portion has a height less than the limited height, frictional force between the needle and the inner wall of the hollow portion 3a is low, whereby the effect of increasing coupling force is insignificant, and that, in the case in which the protruding portion has a height greater than the limited height, the needle 10 may not be easily inserted into the hollow portion 3a when inserted into the hollow portion 3a in an interference fitting fashion.

In addition, the groove portions 110 formed in the insertion region D1 need not necessarily have the same slope. If necessary, the groove portions may be formed so as to intersect with each other in an "X" shape as well as an oblique shape.

Furthermore, in the present invention, the depth D2 of the groove portion 110 and the height H of the protruding portion 120 are calculated on the basis of the surface of the needle 10, and the width W of the groove portion 110 means the distance between a pair of the protruding portions 120 that are joined to each other so as to face each other at the upper end of the groove portion 110.

Moreover, in the present invention, the coupling means 100 may further include an auxiliary fixture 130, wherein the auxiliary fixture 130 is formed as the result of melting of the needle 10 melted to form the groove portion 110 being fixed on the surface of the needle 10 in the form of particles (see FIG. 5).

That is, in the case in which the groove portion 110 is formed through a laser applied to the outer surface of the needle 10 at a predetermined temperature, a portion of the needle that is melted and splashed by the temperature is formed on the surface of the needle 10 in a nonspecific pattern. The melted portion is temporarily maintained at a high temperature, and therefore the melted portion is fixed to the surface of the needle 10, which is made of a metal material or a stainless material, to form the auxiliary fixture 130.

The auxiliary fixture 130 increases the frictional force with the inner wall of the hollow portion 3a together with the protruding portion 120 in order to increase coupling force and fixing force between the needle 10 and the expansion portion 3.

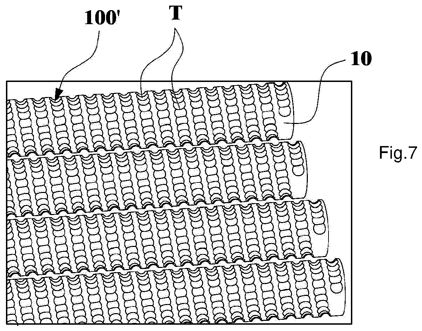

Meanwhile, as shown in FIG. 7, a coupling means 100' according to another embodiment of the present invention may be configured such that a laser is applied to the outer circumferential face of the needle 10 corresponding to the insertion region in a point shot fashion and such that the surface of the needle abutting the laser is distorted by the temperature of the laser, whereby the entire surface of the insertion region is roughened by melting and therefore the needle is fixed due to the rough texture of the coupling means when inserted into the expansion portion 3.

In addition, as shown, the coupling means 100', formed by applying the laser to the surface of the needle 10 in the point shot fashion, may be formed along the outer circumferential surface of the insertion region of the needle 10 in the form of a screw, and contact marking portions T, which directly contact the laser, may be arranged so as to neighbor each other while being spaced apart from each other by a predetermined distance in the longitudinal direction of the insertion region, whereby the coupling means 100' may have a rough texture.

In the cannula 1 according to the present invention having the above-described construction, the needle 10, configured such that the adhesive G is applied over the insertion region D1 in which the coupling means 100 including the groove portion 110 and the protruding portion 120 and further including the auxiliary fixture 130 is formed, is inserted into the expansion portion 3, whereby the present invention has the effect of increasing coupling force and fixing force between the expansion portion 3 and the needle 10 due to frictional force of the coupling means 100, unlike the conventional art.

### [Industrial Applicability]

Although the present invention has been described with reference to the specific particulars, such as concrete components, the limited embodiments, and the drawings, as described above, these are provided only for overall understanding of the present invention, the present invention is not limited to the above embodiments, and it may be apparent to a person having ordinary skill in the art to which the present invention pertains that various modifications and alterations are possible from the above description.

Therefore, the idea of the present invention is not limited to the described embodiment, and the appended claims and all equal or equivalent modifications to the claims fall into the category of the idea of the present invention.

## Claims

1. A cannula for medical treatment configured such that an expansion portion and a needle are adhered to each other via an adhesive, the cannula having excellent coupling force of the needle, wherein
the needle (10) is formed so as to have a cylindrical tubular shape or a cylindrical shape having a predetermined length, and comprises a coupling means (100) formed at an outer surface thereof to increase coupling force due to frictional force with the expansion portion (3) when the needle is inserted into and adhered to the expansion portion (3), and wherein
the coupling means (100) comprises: a groove portion (110) formed by melting an outer surface of the needle (10) through laser machining so as to have a predetermined depth in an insertion region (D1) of the needle (10), which is inserted into the expansion portion (3); a protruding portion (120) protruding in an outward direction of the needle (10) so as to be connected to the groove portion (110); and an auxiliary fixture (130) formed on the outer surface of the needle (10) so as to be adjacent to the groove portion (110) while having a nonspecific pattern, the auxiliary fixture being formed as a result of melting generated at a time of laser machining of the groove portion (110) being fixed to the outer surface of the needle (10).

2. The cannula according to claim 1, wherein the groove portion (110) has a depth (D2) of 1 to 60 µm and a width (W) of 20 to 100 µm.

3. The cannula according to claim 1, wherein the protruding portion (120) has a height (H) of 1 to 30 µm.
